(19) Europäisches Patentamt
European Patent Office
Office européen des brevets

(11) **EP 2 248 797 A1**

(12) **EUROPEAN PATENT APPLICATION**
published in accordance with Art. 153(4) EPC

(43) Date of publication:
**10.11.2010 Bulletin 2010/45**

(21) Application number: **09715728.3**

(22) Date of filing: **26.02.2009**

(51) Int Cl.:
*C07C 53/126* [(2006.01)]       *B22F 9/30* [(2006.01)]
*C07C 51/02* [(2006.01)]

(86) International application number:
**PCT/JP2009/053559**

(87) International publication number:
**WO 2009/107721 (03.09.2009 Gazette 2009/36)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **29.02.2008 JP 2008050988**
**29.02.2008 JP 2008050989**
**17.11.2008 JP 2008293168**

(71) Applicant: **Toyo Seikan Kaisha, Ltd.**
**Tokyo 100-8522 (JP)**

(72) Inventors:
• **OHASHI, Kazuaki**
**Yokohama-shi**
**Kanagawa 240-0062 (JP)**

• **SATO, Kazuhiro**
**Yokohama-shi**
**Kanagawa 240-0062 (JP)**
• **KASAI, Anzu**
**Yokohama-shi**
**Kanagawa 240-0062 (JP)**
• **HIRATSUKA, Daisuke**
**Osaka-shi**
**Osaka 531-8526 (JP)**
• **SUZUKI, Shigeru**
**Osaka-shi**
**Osaka 531-8526 (JP)**

(74) Representative: **Manley, Nicholas Michael**
**W.P. Thompson & Co.**
**Coopers Building**
**Church Street**
**Liverpool L1 3AB (GB)**

(54) **FATTY ACID METAL SALT FOR FORMING ULTRAFINE METAL PARTICLE**

(57) A fatty acid metal salt used for forming ultrafine metal particles satisfying at least one of that:
(i) the water content is 200 ppm or less;
(ii) the volume-cumulative particle diameter D90 is 80 μm or smaller as measured by the particle size distribution measuring method of the laser diffraction/scattering type; or
(iii) a metal of an atomic weight of 50 to 200 is contained, and the amount of the unreacted substance or the by-product is 4.0 mol% or less when the fatty acid metal salt is formed. The fatty acid metal salt can be favorably used for forming ultrafine metal particles in a resin, or for forming a resin composition, a coating, a dispersion solution or a molded article containing the ultrafine metal particles.

EP 2 248 797 A1

**Description**

Technical Field:

This invention relates to a fatty acid metal salt. More specifically, the invention relates to a fatty acid metal salt that can be favorably used as a precursor for forming ultrafine metal particles, a resin composition containing the ultrafine metal particles, a coating containing the ultrafine metal particles or a dispersion solution of the ultrafine metal particles.

Background Art:

Fatty acid metal salts have heretofore been widely used in many fields such as in the field of electronic printing, in the field of powder metallurgy, in the field of cosmetics, in the field of coating materials, in the field of resin working and so on. For example, magnesium salts and calcium salts of fatty acid have been used for improving luster of the skin and adhesiveness in the field of cosmetics and for improving dispersion of pigments in the field of resin working. On the other hand, a silver salt of fatty acid has been used as a material for thermally developing and recording images in the photoengraving process and in the medical use. In recent years, however, the silver salt of fatty acid has been used as a precursor for obtaining ultrafine metal particles having an average particle diameter of 1 to 100 nm as disclosed in patent documents 1 and 2 described below.

That is, according to the patent document 1 described below, an organometal compound such as fatty acid silver or fatty acid gold salt is thermally decomposed by the solid phase reaction in an inert gas atmosphere to synthesize ultrafine metal particles of silver or gold having an average particle diameter of 1 to 100 nm of which the surfaces are protected by the fatty acid. According to the patent document 2, on the other hand, a mixture of silver or gold salt of a fatty acid and a resin is heat-molded at a temperature higher than a temperature at which the fatty acid metal salt starts thermally decomposing but lower than a temperature at which the resin thermally deteriorates to form ultrafine metal particles having an average particle diameter of 1 to 100 nm in a molded article of resin.

Such ultrafine metal particles exhibit a singular property different from those of a bulk, and study has been forwarded in an attempt to use them in a variety of fields, such as using them for ink-jet materials, recording materials and catalysts, using them as a material of electronic devices like electrically conducting paste, using them as a coloring material by utilizing their plasmon absorption, etc. Study has, further, been forwarded in an attempt to put resin molded articles in which the ultrafine metal particles are stably dispersed into a wide range of use as the electrically conducting materials, magnetic materials and electromagnetic wave-absorbing materials.

It has, further, been clarified by the present applicant that a resin compound containing ultrafine metal particles of which the surfaces are modified with an organic acid produced by, for example, the method of the patent document 2 has property for adsorbing offensively smelling components such as methyl mercaptane and volatile organic compounds (hereinafter "VOCs") such as formaldehyde, and has antibacterial property as well as properties for inactivating microproteins such as allergenic substances (patent documents 3 and 4).

Study has been forwarded to utilize the above-mentioned ultrafine metal particles in a variety of fields. As the production method of obtaining such ultrafine metal particles, there can be, usually, exemplified a gas phase method in which a vapor of a metal vaporized at a high temperature is fed into a gas phase so as to collide with the molecules of gas, followed by quick quenching to form fine particles and a liquid phase method in which a reducing agent is added to a solution containing metal ions to reduce the metal ions. However, a method in which a fatty acid metal salt is mixed as a precursor into the resin and the resin is heat-molded, is a generally employed and richly productive method making it possible to very simply obtain a resin composition containing ultrafine metal particles featuring narrow particle size distribution and excellent dispersion stability.

There have been known the following two representative methods of producing fatty acid metal salts.

The first production method is a melting method in which a fatty acid is heated and melted and is, thereafter, reacted with an oxide or a hydroxide of a metal to form a fatty acid metal salt. This method features a simple production facility accompanied, however, by such defects that it is difficult to perfectly execute the reaction permitting the reaction product to easily stay in the formed product and, further, necessitating a step of pulverizing and finely granulating the formed product.

The second production method is a double decomposition method in which a fatty acid is saponified with a hydroxide of an alkali metal such as sodium hydroxide, or an aqueous solution containing metal ions is added to an aqueous solution prepared by dissolving a fatty acid alkali metal salt therein to form a fatty acid metal salt. This reaction can be conducted without requiring high-temperature conditions, and the after-treatments can be conducted through simple steps such as filtering, washing, drying and milling, offering such an advantage that particles of small sizes can be obtained. Therefore, this method has been frequently utilized for the commercial productions.

Patent document 1: JP-A-10-183207

Patent document 2: JP-A-2005-348213

Patent document 3: WO2008/29932
Patent document 4: WO2008/69034

Disclosure of the Invention:

Problems that the Invention is to Solve:

It is considered that either the melting method or the double decomposition method can be adapted as the method of producing a fatty acid metal salt for use as a precursor of the ultrafine metal particles. However, it has not been known yet concerning the fatty acid metal salt of what kind of properties can be more favorably used for forming ultrafine metal particles, a resin composition containing ultrafine metal particles or a molded article thereof.
Therefore, the object of the present invention is to provide a fatty acid metal salt that can be favorably used for forming ultrafine metal particles in a resin, or for forming a resin composition, a coating, a dispersion solution or a molded article containing ultrafine metal particles.

Means for Solving the Problems:

According to the present invention, there is provided a fatty acid metal salt used for forming ultrafine metal particles satisfying at least one of that:

(i) the water content is 200 ppm or less;
(ii) the volume-cumulative particle diameter D90 is 80 $\mu$m or smaller as measured by the particle size distribution measuring method of the laser diffraction/scattering type; or
(iii) a metal of an atomic weight of 50 to 200 is contained, and the amount of the unreacted substance or the by-product is 4.0 mol% or less when the fatty acid metal salt is formed.

In the fatty acid metal salt of the present invention, it is desired that:

1. The ultrafine metal particles have an adsorptive property and/or a property for inactivating microproteins;
2. The metal is at least the one selected from the group consisting of Cu, Ag, Au, Id, Pd, Pt, Fe, Ni, Co, Zn, Nb, Ru and Rh;
3. The metal is a combination of at least the one selected from the group consisting of Cu, Au, Id, Pd, Pt, Fe, Ni, Co, Zn, Nb, Ru and Rh, and Ag;
4. The fatty acid has 3 to 30 carbon atoms.

According to the present invention, further, there is provided a method of producing a resin composition containing ultrafine metal particles, comprising heating the fatty acid metal salt at a temperature at which the fatty acid metal salt thermally decomposes in a resin but lower than a temperature at which the resin is deteriorated so that ultrafine metal particles are formed from the fatty acid metal salt in the resin and are dispersed therein.
According to the present invention, further, there is provided a method of producing a coating containing ultrafine metal particles, comprising heating the fatty acid metal salt at a temperature at which the fatty acid metal salt thermally decomposes in the coating but lower than a temperature at which the coating component is deteriorated so that ultrafine metal particles are formed from the fatty acid metal salt in the coating and are dispersed therein.
According to the present invention, further, there is provided a method of producing a dispersion medium containing ultrafine metal particles, comprising heating the fatty acid metal salt at a temperature at which the fatty acid metal salt thermally decomposes in the dispersion medium but lower than a boiling point of the dispersion medium so that ultrafine metal particles are formed from the fatty acid metal salt in the dispersion medium and are dispersed therein.
The present inventors have discovered that a resin composition, a coating or a dispersion solution having good color tone and having particularly excellent ability for adsorbing offensively smelling substances and VOCs, antibacterial property, and ability for inactivating microproteins, can be provided by mixing and heating a fatty acid metal salt that satisfies at least one of that:

(i) the water content is 200 ppm or less;
(ii) the volume-cumulative particle diameter D90 is 80 $\mu$m or smaller as measured by the particle size distribution measuring method of the laser diffraction/scattering type; or
(iii) a metal of an atomic weight of 50 to 200 is contained, and the amount of the unreacted substance or the amount the by-product is 4.0 mol% or less when the fatty acid metal salt is formed;
in a resin, a coating material or a dispersion medium.

(Water content of the fatty acid metal salt)

The above-mentioned actions and effects exhibited by the fatty acid metal salt having a water content of 200 ppm of the invention will also become obvious from Examples appearing later.

That is, in Examples appearing later, films were formed by using fatty acid metal salts having different water contents and were measured for their absorbencies by using a spectrophotometer (manufactured by Shimazu Seisakusho Co.). It has been known that ultrafine particles of a noble metal or copper develop a color due to plasmon absorption that occurs as free electrons receive oscillation in a photomagnetic field. The wavelength of absorption is specific to the kind of the metal. In the case of ultrafine silver particles, the absorption occurs near a wavelength of 420 nm.

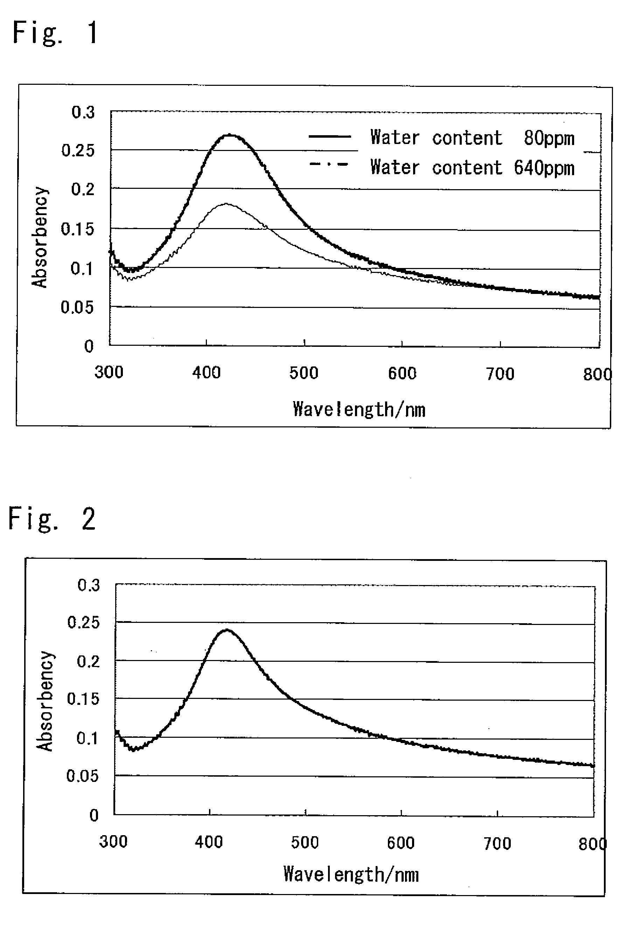

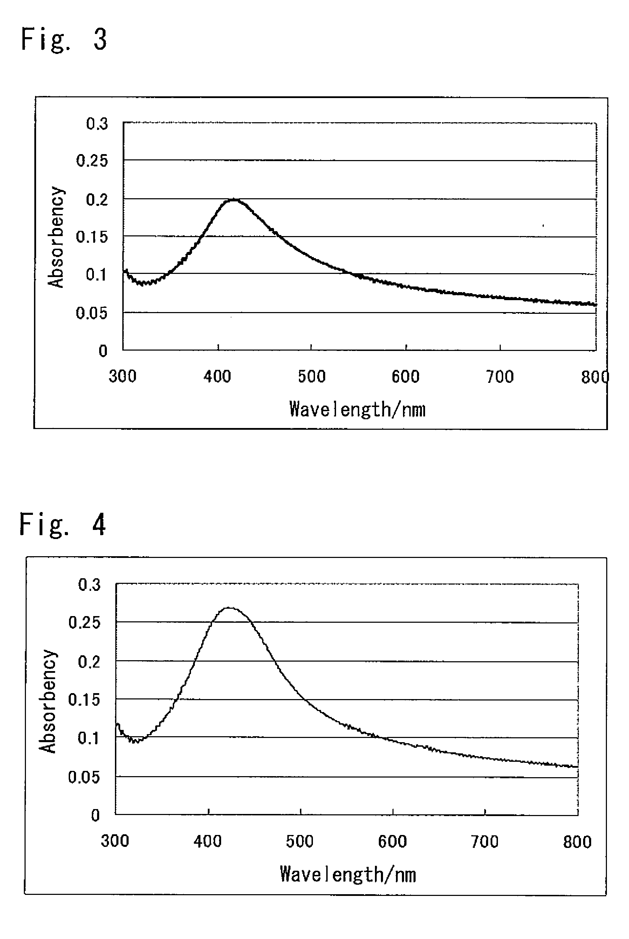

From Figs. 1 to 5, it will be learned that the films of Examples 1 to 4 and Comparative Examples 1 and 2 have absorption near 420 nm due to plasmon absorption of silver. From Figs. 1 to 4, further, it will be learned that the films obtained by using silver stearate having a small water content have larger absorbencies near 420 nm.

These results indicate that use of the fatty acid metal salt having a water content of less than 200 ppm makes it possible to form ultrafine silver particles having a narrow particle size distribution being stably dispersed without aggregated in the molded articles. It is obvious that the molded articles, further, exhibit superior adsorption performance to those obtained by using fatty acid metal salts having water contents larger than 200 ppm.

(Particle size distribution of fatty acid metal salt)

The fatty acid metal salt having a large particle diameter could become a cause of forming fine metal particles having very large particle diameters when they are mixed and heated in a resin or in a dispersion medium. As a result, the resin composition or the dispersion solution exhibits very decreased ability for adsorbing offensively smelling substances, very decreased antibacterial property or very decreased ability for inactivating microproteins.

On the other hand, the fatty acid metal salt of the present invention has a particle diameter D90 with which the volume cumulation becomes 90% of not larger than 80 $\mu$m, particularly, the particle diameter D90 of not larger than 80 $\mu$m and a volume-cumulative average particle diameter D50 of not larger than 30 $\mu$m, and is capable of efficiently expressing the above-mentioned action and effect.

In the particle size distribution measuring method of the laser diffraction/scattering type, an aggregated particle, too, is counted as a particle. According to the present invention in which the particle diameter D90 with which the volume cumulation becomes 90% is not larger than 80 $\mu$m, particularly, the particle diameter D90 is not larger than 80 $\mu$m and the particle diameter D50 with which the volume cumulation becomes 50% is not larger than 30 $\mu$m, therefore, it is meant that particles having small particles diameters are distributed containing little aggregated particles.

The above-mentioned actions and effects exhibited by the fatty acid metal salt having the above particle diameter distribution of the invention will also become obvious from the results of Examples appearing later.

That is, in Examples appearing later, films were formed by using fatty acid metal salts having different volume-cumulative average particle diameters D90 and D50 and were measured for their absorbencies by using a spectrophotometer (manufactured by Shimazu Seisakusho Co.). In the case of ultrafine silver particles as described above, the absorption is exhibited near a wavelength of 420 nm.

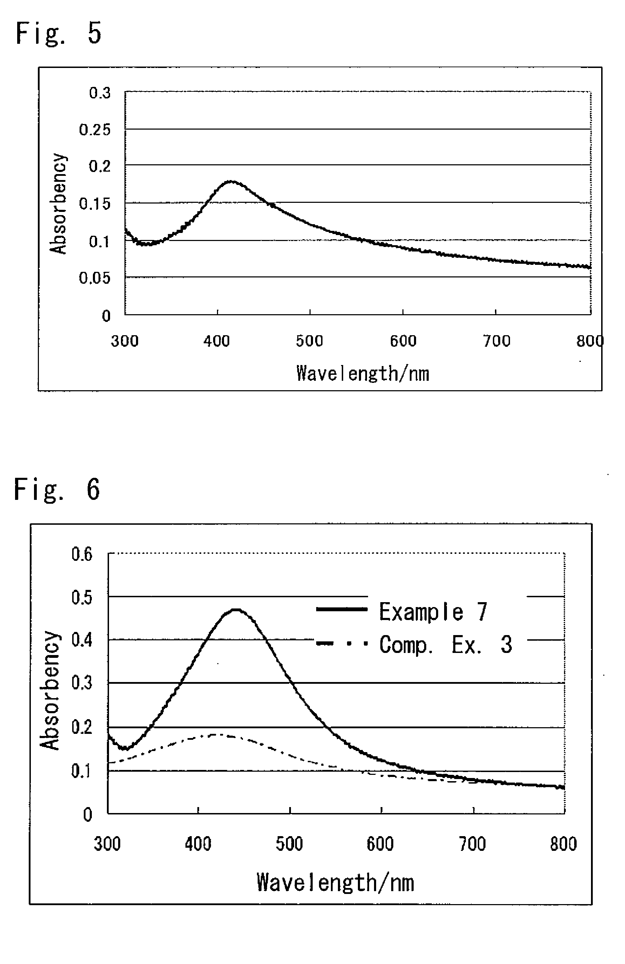

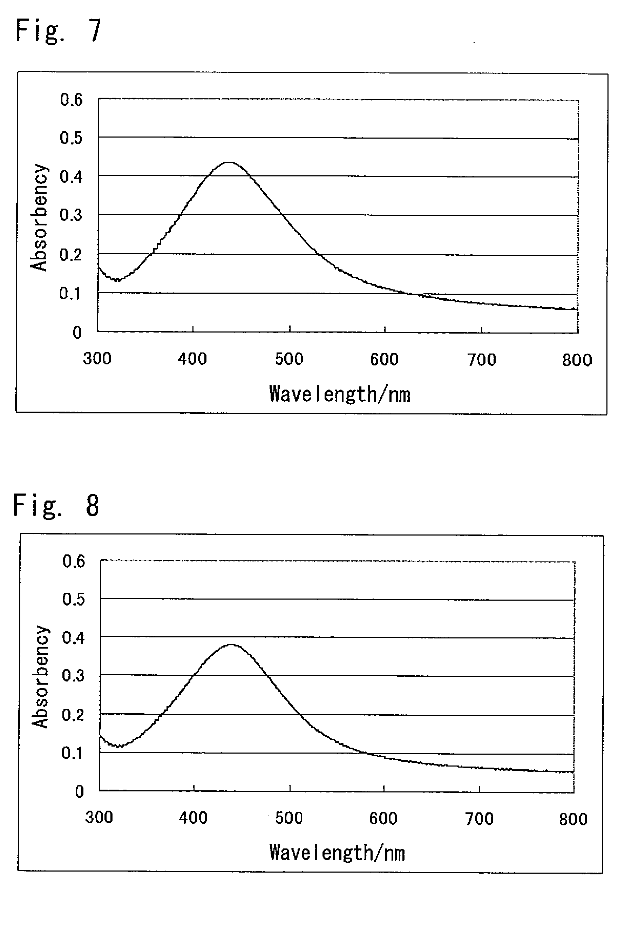

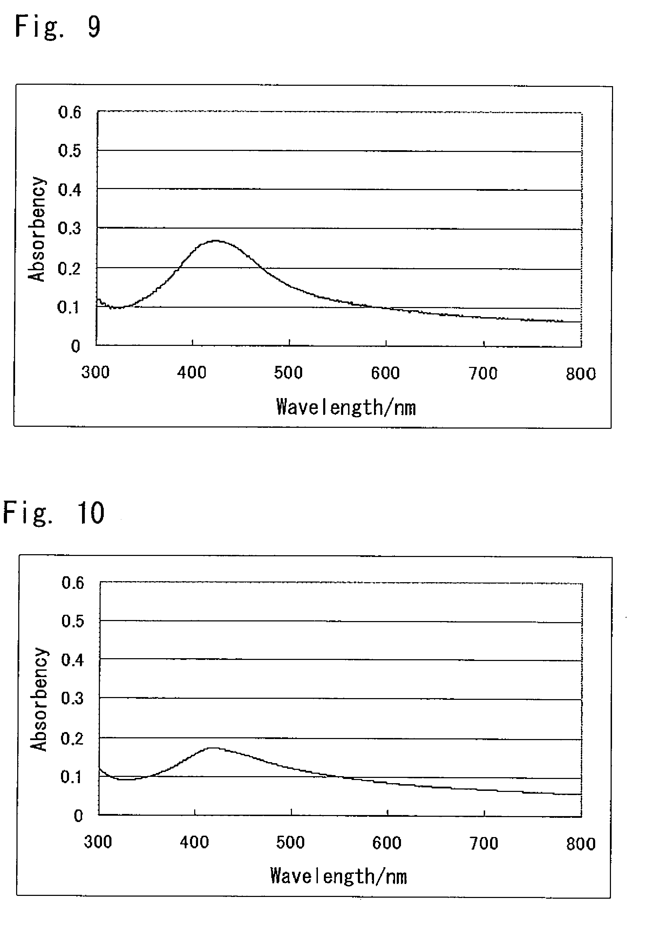

From Figs. 6 to 10, it will be learned that the films of Examples 7 to 10 and Comparative Examples 3 and 4 have absorption near 420 nm due to plasmon absorption of silver. From Figs. 6 to 9, further, it will be learned that the films obtained by using silver stearates having small D90 and D50 exhibit larger absorbencies near 420 nm.

These results indicate that use of the fatty acid metal salt having a particle diameter D90 of not larger than 80 $\mu$m makes it possible to form ultrafine silver particles having a narrow particle diameter distribution being homogeneously dispersed without aggregated in the molded articles. It is obvious that the molded articles exhibit superior adsorption performance to those obtained by using fatty acid metal salts having particle diameters larger than the value of particle diameter D90 specified by the present invention.

(Amount of the unreacted substance or the by-product in the fatty acid metal salt)

As described earlier, a melting method and a double decomposition method have been known as representative methods of producing fatty acid metal salts. According to the melting method, a fatty acid is heated and melted, and is, thereafter, reacted with an oxide or a hydroxide of a metal to form a fatty acid metal salt. As a starting material, use is made of a fatty acid obtained by dissolving an alkali such as sodium hydroxide in water, or an alkali metal salt of a fatty acid. With the melting method, however, it is difficult to perfectly execute the reaction. Therefore, unreacted products tend to remain; i.e., alkali metal salts of fatty acid remain as unreacted products and alkali metal salts remain as by-products in the formed fatty acid metal salt.

According to the double decomposition method, on the other hand, a fatty acid is saponified with a hydroxide of an alkali metal such as sodium hydroxide, or an aqueous solution containing metal ions is added to an aqueous solution prepared by dissolving a fatty acid alkali metal salt or a fatty acid ammonium salt therein to form a fatty acid metal salt. Therefore, alkali metal salts of fatty acid or fatty acid ammonium salts remain as unreacted products, or alkali metal salts or ammonium salts remain as by-products in the formed fatty acid metal salt.

According to the present invention, it was discovered that among the above unreacted products and by-products, if the alkali metal salt or the ammonium salt such as sodium salt or potassium salt is present in an amount of not less than 4.0 mol% in the fatty acid metal salt, the ultrafine metal particles are formed very poorly efficiently, and that the resin composition, coating material or dispersion solution exhibits decreased adsorption performance or decreased effect for inactivating microproteins.

Table 3 tells that the plates of Examples 11 to 15 have mercaptane deodorizing ratios higher than those of the plates of Comparative Examples 5 and 6. It is, therefore, learned that the plates obtained by using silver stearates containing sodium salt, calcium salt and ammonium salt in small amounts, have higher mercaptane deodorizing ratios.

Effects of the Invention:

If ultrafine metal particles are formed in the resin, in the coating material or in the dispersion medium by using, as a precursor, a fatty acid metal salt that satisfies at least one of that:

(i) the water content is 200 ppm or less;
(ii) the volume-cumulative particle diameter D90 is 80 $\mu$m or smaller as measured by the particle size distribution measuring method of the laser diffraction/scattering type; or
(ii) a metal of an atomic weight of 50 to 200 is contained, and the amount of the unreacted substance or the by-product is 4.0 mol% or less when the fatty acid metal salt is formed;
then it becomes possible to stably obtain ultrafine metal particles having an average particle diameter of 1 to 100 nm.

Further, the resin molded article, coating or dispersion solution containing the ultrafine metal particles exhibits very excellent performance such as ability for adsorbing offensively smelling substances and VOCs, antibacterial property and ability for inactivating microproteins as compared to the cases of when there are used fatty acid metal salts of which the water content, particle size distribution or the content of unreacted substance or by-product does not lie in the ranges specified by the present invention.

The fatty acid metal salt of the present invention can be favorably used as a precursor for forming ultrafine metal particles, or for forming a resin composition, a coating or a dispersion solution containing ultrafine metal particles.

The resin molded article containing ultrafine metal particles, the coating containing ultrafine metal particles or the dispersion solution containing ultrafine metal particles formed by using the fatty acid metal salt of the present invention, is capable of effectively adsorbing smelling components and VOCs, capable of expressing excellent deodorizing performance or VOC-adsorbing performance, capable of effectively inactivating cedar pollen, allergenic substance stemming from tick, enzymes and microproteins such as viruses. Besides, ultrafine metal particles can be formed and homogeneously dispersed simultaneously with the mold working, enabling excellent productivity to be realized.

Brief Description of the Drawings:

[Fig. 1] is a graph showing absorbencies of molded articles to which silver stearates are added of Example 1 and Comparative Example 1.
[Fig. 2] is a graph showing absorbency of a molded article to which silver stearate is added of Example 2.
[Fig. 3] is a graph showing absorbency of a molded article to which silver stearate is added of Example 3.
[Fig. 4] is a graph showing absorbency of a molded article to which stearates of silver and cupper are added of Example 4.
[Fig. 5] is a graph showing absorbency of a molded article to which silver stearate is added of Comparative Example 1.
[Fig. 6] is a graph showing absorbency of a molded article to which silver stearate is added of Example 7 and absorbency of a molded article to which silver stearate is added of Comparative Example 3.
[Fig. 7] is a graph showing absorbency of a molded article to which silver stearate is added of Example 8.
[Fig. 8] is a graph showing absorbency of a molded article to which silver stearate is added of Example 9.
[Fig. 9] is a graph showing absorbency of a molded article to which stearates of silver and copper are added of Example 10.
[Fig. 10] is a graph showing absorbency of a molded article to which silver stearate is added of Comparative Example 4.
[Fig. 11] is a diagram showing a relationship between the contents of sodium or potassium and the deodorizing ratios of Table 3.

Mode for Carrying Out the Invention:

(Fatty acid metal salts)

The kind of metal in the fatty acid metal salt of the present invention is at least the one selected from the group consisting of Cu, Ag, Au, Id, Pd, Pt, Fe, Ni, Co, Zn, Nb, Ru and Rh. Particularly, Cu, Ag, Co and Ni are desired from the standpoint of their high deodorizing and antibacterial performance. Further, a plurality of metals may be contained. In this case, it is desired that Ag is used as an essential component in combination with at least one of other metals.

Further, the fatty acid in the fatty acid metal salt of the present invention is a fatty acid having 3 to 30 carbon atoms, which may be saturated or unsaturated. Examples thereof include caproic acid, caprylic acid, capric acid, lauric acid, myristic acid, palmitic acid, oleic acid, linoleic acid, linolenic acid, stearic acid and arachidinic acid. There may be used a plurality of fatty acids.

Desirably, a straight-chain saturated fatty acid having 12 to 22 carbon atoms is used. If the number of carbon atoms is smaller than 12, the fatty acid highly dissolves in water, and the yield of the fatty acid metal salt which is the product decreases. If the number of carbon atoms is not smaller than 23, the fatty acid lowly dissolves in water which is the starting material often making it difficult to form a fatty acid metal salt.

The fatty acid metal salt according to the present invention may be produced by either the above-mentioned melting method or the double decomposition method. However, the double decomposition method is preferred from the standpoint of obtaining a fatty acid metal salt having small particle diameters.

Though there is no particular limitation on the starting metal provided it is soluble in water, it is desired to use a nitrate. As the starting material of the fatty acid, there can be used a fatty acid or an alkali metal salt of fatty acid. The fatty acid that is to be used is dissolved in water by using an alkali such as NaOH.

In producing the fatty acid metal salt of the invention, if the valency of the fatty acid used as the starting material is denoted by A, the mol number thereof by X, the valency of metal ions by B and the mol number thereof by Y, then it is desired that the starting materials are blended at a ratio AX/BY of not less than 0. 9 but less than 1.1. If the ratio is less than 0.9, the fatty acid metal salt is formed poorly efficiently. If the ratio is not less than 1.1, the content of metal becomes low and the resin molded article exhibits decreased deodorizing or antibacterial performance.

The reaction medium used for the synthesis may be water or a mixed solution of water and an organic compound. The organic compound that can be mixed is the one that can be blended with water, and is limited to the one that does not destroy the structure of the starting materials or of the formed product. As the organic compound that can be mixed, there can be exemplified alcohols such as methanol, ethanol, propanol, diethylene glycol and polyethylene glycol; glycol ethers such as ethylene glycol monoethyl ether and ethylene glycol monoisopropyl ether; cyclic ethers such as tetrahydrofuran and 1,4-dioxane; carboxylic acids such as acetic acid, propionic acid and butyric acid; ketones such as acetone, methyl ethyl ketone and cyclohexanone; and polar solvents such as dimethyl sulfoxide and dimethyl formamide.

According to the double decomposition method, an aqueous solution of the starting metal and an aqueous solution of the fatty acid are mixed together to obtain a desired fatty acid metal salt.

According to the invention, there is no limitation on the reaction method provided there is obtained a fatty acid metal salt having particle diameters smaller than a predetermined value. For example, to obtain a fatty acid metal salt having small particle diameters and little aggregation, it is desired (1) to intensify the stirring at the time of mixing and (2) to conduct the mixing as quickly as possible in the step of mixing the aqueous solution containing fatty acid and the aqueous solution containing metal ions to form a desired fatty acid metal salt. If the step of reaction is not appropriate, there is formed a fatty acid metal salt having large particle diameters or a fatty acid metal salt that is aggregated to a conspicuous degree.

After the above step of reaction, the slurry containing the obtained fatty acid metal salt is passed through the steps of washing and filtering, and is dried.

In the present invention, it is important to sufficiently wash the obtained fatty acid metal salt in order to effectively decrease the unreacted product and by-product. The washing can be conducted by a known method such as filtration or decantation. The washing may be conducted by using a solvent in which the unreacted product and by-product dissolve but the fatty acid salt does not dissolve. Here, water and ethanol can be preferably used. In conducting the washing, the degree of washing can be estimated relying on the electric conductivity or pH of the washing solution.

There is no particular limitation on the drying method provided the water content of the fatty acid metal salt after drying becomes 200 ppm or less. For instance, there can be used a vacuum drier, a freeze drier or an air stream-type drier.

In the case of the hot-air type drier, it is desired to conduct the drying at a temperature of 80 to 120°C and, particularly, 90 to 110°C. If the temperature is lower than 80°C, water is not sufficiently evaporated. If the temperature is higher than 120°C, on the other hand, the reaction becomes undesirable, such as the fatty acid is partly decomposed by heat. Besides, some particles melt-adhere together causing the particle diameter to increase.

Further, the cake obtained before the drying may be pressed to decrease the amount of water. In this case, the pressing pressure is better small and, desirably, the step of drying is carried out without pressing. An undesirably large pressing pressure could cause the particles to aggregate, which is not desirable.

Further, though the fatty acid metal salt can be controlled for its water content to be 200 ppm or less by drying, the water content increases after the passage of time. It is, therefore, desired that the fatty acid metal salt of the invention is stored under a dry condition shutting off light except when the fatty acid metal salt is used right after the production, i.e., except when the fatty acid metal salt is mixed into the resin which is then heat-molded. Just before the use, the fatty acid metal salt of the invention may be dried again and is used.

(Resin compositions containing ultrafine metal particles)

Upon heat-molding a resin composition blended with the fatty acid metal salt of the invention, there is obtained an ultrafine metal particle-containing resin composition in which the ultrafine metal particles having the above-mentioned actions and effects are homogeneously dispersed.

As the resin to be blended with the fatty acid metal salt of the invention, there can be used any known thermoplastic resin that can be melt-molded, like olefin resins such as low-, intermediate- or high-density polyethylene, linear low-density polyethylene, linear ultra-low-density polyethylene, isotactic polypropylene, syndiotactic polypropylene, propylene/ethylene copolymer, polybutene-1, ethylene/butene-1 copolymer, propylene/butene-1 copolymer and ethylene/propylene/butene-1 copolymer; polyester resins such as polyethylene terephthalate, polybutylene terephthalate and polyethylene naphthalate; polyamide resins such as nylon 6, nylon 6,6 and nylon 6,10; and polycarbonate resin.

According to the present invention, it is particularly desired to use polyethylene, polypropylene or polyester for the resin composition containing ultrafine metal particles.

Depending on the use, further, the resin composition containing ultrafine metal particles of the invention can be blended with various blending agents that have been known per se. such as filler, plasticizer, leveling agent, viscosity-imparting agent, viscosity-reducing agent, stabilizer, antioxidant and ultraviolet ray absorber according to known recipe.

It is desired that the fatty acid metal salt of the invention is added in an amount of 0.001 to 5 parts by weight per 100 parts by weight of the resin. If the amount is smaller than the above range, the effect possessed by the ultrafine metal particles is not obtained to a sufficient degree. If the amount is larger than the above range, on the other hand, the ultrafine metal particles aggregate and cannot be homogeneously dispersed, which is not desirable.

According to the present invention, the resin composition can be subjected to a known melt molding such as two-roll method, injection molding, extrusion molding or compression molding to finally obtain the resin-molded articles in shapes that meet the use, such as granules, pellets, fibers, films, sheets, containers, etc.

The temperature for molding the resin composition containing ultrafine metal particles varies depending on the molding method and the kinds of the resin and the fatty acid metal salt that are used, and cannot be definitely defined. It is, however, necessary that the resin composition is heat-molded at a temperature at which the resin that is used is not thermally deteriorated but the fatty acid metal salt is thermally decomposed in the resin. Further, the heat-treating conditions are affected by the heat of shearing due to the screw or by the residence time in addition to the setpoint temperature of the working machine such as the extruder or the molding machine. Therefore, it is desired to adjust the working conditions such as residence time, rotational speed of the screw, etc. depending on the setpoint temperature in the above temperature range.

Further, the molded article of the resin composition containing ultrafine metal particles of the invention may by itself constitute a resin molded article but may also assume a multi-layer structure in combination with other resins.

(Coatings containing ultrafine metal particles)

A coating containing the above ultrafine metal particles can be formed by using a coating agent prepared by using the fatty acid metal salt of the invention.

That is, as described above, through the heat treatment at the time of firing the coating, the fatty acid metal salt of the invention forms ultrafine metal particles that are homogeneously dispersed in the coating component; i.e., ultrafine metal particles are made present in the coating.

It is desired that the fatty acid metal salt is added in an amount of 0.001 to 5 parts by weight per 100 parts by weight of the coating component. If the amount is smaller than the above range, the effect possessed by the ultrafine metal particles is not expressed to a sufficient degree. If the amount is larger than the above range, on the other hand, the ultrafine metal particles may aggregate, which is not desirable.

There can be used various kinds of coating components for containing the fatty acid metal salt of the invention provided they are capable of foaming a coating by heating. For example, though not limited thereto only, there can be used known coating compositions such as acrylic coating material, epoxy coating material, phenol coating material, urethane coating material, polyester coating material and alkyd resin coating material.

Depending on the use, further, the coating agent for forming the coating, too, can be blended with various blending agents that have been known per se. such as leveling agent, viscosity-imparting agent, viscosity-reducing agent, stabilizer, antioxidant, ultraviolet ray absorber and coloring agent according to known recipe like the case of the molded articles. The heat-treating conditions for forming the coating may vary depending on the coating component and the kind of the fatty acid metal salt that are used, and cannot be definitely defined. It is, however, necessary that the heat treatment is conducted at a temperature at which the coating component that is used is not thermally deteriorated and the fatty acid metal salt is thermally decomposed in the resin for 60 to 600 seconds.

(Ultrafine metal particle-dispersed solutions)

A dispersion solution is prepared by using the fatty acid metal salt of the invention. Namely, the fatty acid metal salt of the invention is dispersed in a dispersion medium and is heated at a temperature higher than a temperature at which the fatty acid metal salt thermally decomposes in the dispersion medium but lower than a boiling point of the dispersion medium to prepare the dispersion solution containing ultrafine metal particles dispersed in the dispersion medium.

As the dispersion medium used for the method of producing a dispersion solution of the invention, a polyhydric alcohol can be preferably used. It is desired that the polyhydric alcohol has a boiling point higher than a temperature at which the fatty acid metal salt thermally decomposes in the dispersion medium, and its examples include polyethylene glycol, diethylene glycol and glycerol. Among them, however, polyethylene glycol is particularly preferably used. Preferably, the polyethylene glycol has an average molecular weight in a range of 200 to 20000 and, particularly, 400 to 10000. Further, a plurality of kinds of those having different molecular weights may be used being mixed together.

In the method of producing the dispersion solution of the invention, it is desired to add the fatty acid metal salt in an amount of $1 \times 10^{-6}$ to 20% by weight and, particularly, $1 \times 10^{-5}$ to 10% by weight to the dispersion solution. If the amount of the fatty acid metal salt is smaller than the above range, the ultrafine metal particles cannot be dispersed in sufficient amounts. If the amount thereof is larger than the above range, on the other hand, the ultrafine metal particles may aggregate.

It is, further, desired to add an antioxidant as a protection agent. Upon adding the antioxidant, thermal deterioration can be prevented at the time of heating.

Though not limited thereto only, examples of the antioxidant that is used include tocopherols (vitamine E), hindered phenol-type antioxidant, phosphorus-type antioxidant and ethylene bisstearic acid amide that have heretofore been known. Particularly desirably, IRGANOX 1010 (registered trade mark) can be used. The antioxidant is desirably added to the dispersion solution in an amount of 0.1 to 10% by weight.

In the method of producing the dispersion solution of the invention, the fatty acid metal salt is added to the dispersion medium and, as required, an antioxidant is added thereto. Thereafter, the mixture is stirred while being heated at a temperature at which the fatty acid metal salt undergoes the thermal decomposition in the dispersion medium but lower than the boiling point of the dispersion medium so that ultrafine metal particles are formed in the dispersion medium. The heating time differs depending upon the kind of the solution that is used and the amount of the fatty acid metal salt that is added, and cannot be definitely defined, but is desirably 1 to 1800 seconds and, particularly, 5 to 300 seconds. After heated and mixed, the dispersion solution is cooled down to room temperature and is filtered. Thus, free fatty acids are removed from the dispersion solution, and there is obtained the dispersion solution in which ultrafine metal particles of the invention and, particularly, ultrafine metal particles having an average particle diameter of 1 to 100 nm are homogeneously dispersed in the dispersion medium.

The dispersion solution obtained by the production method of the invention can by itself be used as an adsorptive (deodorant) or a microprotein-inactivating agent but is, desirably, used being diluted with a solvent.

The solvent used for the dilution may be, though not limited thereto only, water such as purified water or ion-exchanged water; lower alcohols such as methanol, ethanol, propanol, isopropanol and butanol; general modified alcohols such as those modified with methanol, those modified with benzole, those modified with triol, those modified with methyl ethyl ketone, those modified with denatonium benzoate and those modified with perfume; modified alcohols such as ethylene glycol monoethyl ether, chloroform, diethyl carbonate, ethyl acetate, ethyl propionate, ethyl butyrate, hexane, and ethyl ether for industrial use; and glycol-type solvents such as ethylene glycol monobutyl ether, diethylene glycol monobutyl ether, propylene glycol monomethyl ether, propylene glycol monopropyl ether, propylene glycol monobutyl ether, propylene glycol diethylene glycol monobutyl ether, dipropylene glycol ethylene glycol monobutyl ether, ethylene glycol monophenyl ether, and triethylene glycol monophenyl ether. These solvents may be used alone or in a combination of two or more kinds.

The present invention preferably uses a low-boiling solvent having a boiling point of not higher than 100°C, such as water or ethanol and, particularly preferably, uses an aqueous solution containing ethanol at a concentration of 1 to 30%. The dispersion solution of the invention can be used by being sprayed or applied onto, or having been soaked in, the dwelling-related members such as floors, walls, curtains, carpets, etc., fibrous products such as of air conditions, woven fabrics, nonwoven fabrics, etc., and the filtering members such as masks, filters, etc.

EXAMPLES

The invention will now be described in further detail with reference to Examples and Comparative Examples to which only, however, the invention is in no way limited.

1. Films containing fatty acid metal salts.

Stearates obtained in Examples 1 to 10 and Comparative Examples 1 to 4 described below were added each in an amount of 0.5% by weight to a low-density polyethylene (manufactured by Sumitomo Kagaku Co.) which was then heat-melted in a biaxial extruder (manufactured by Toyo Seiki Co.) set at a temperature of 250°C, extruded through a T-die film-forming machine (manufactured by Toyo Seiki Co.), and was wound on a take-up roll. Thus, films containing fatty acid metal salts and having a thickness of 50 $\mu$m were obtained.

2. Measuring the water contents of fatty acid metal salts.

The fatty acid metal salts obtained in Examples 1 to 10 and in Comparative Examples 1 to 4 were measured for their water contents by using the Karl Fischer's water content meter (manufactured by Dian Instruments Co.).

3. Measuring the deodorizing ratios of films containing ultrafine metal particles.

(1) Measuring the methyl mercaptane concentrations of when not deodorized.

By using a micro syringe, 5 $\mu$L of offensively smelling methyl mercaptane were injected into 500-mL glass bottles purged with a nitrogen gas and sealed for their mouth portions with rubber plugs, were so adjusted that their concentrations were 10, 20 ppm, and were left to stand at room temperature (25°C) for a whole day. After left to stand for a whole day, detector tubes manufactured by Gas-Tech Co. were inserted in the bottles to measure the concentrations of the remaining methyl mercaptane, which were regarded as the concentrations (A) of methyl mercaptane of when not deodorized.

(2) Measuring the methyl mercaptane concentrations after deodorized.

Films containing ultrafine metal particles obtained in Examples 1 to 10 and in Comparative Examples 1 to 4 were each cut into a 5-cm square shape, and were hung in the 500-mL glass bottles using a resin yarn. A rotor was introduced for stirring, and the interiors of the bottles were purged with nitrogen. Thereafter, by using a micro syringe (manufactured by Ito Seisakusho Co.), 5 $\mu$L of an aqueous solution of offensively smelling methyl mercaptane was added thereto dropwise.
Next, by using a stirrer, the methyl mercaptane aqueous solution was stirred for 15 minutes so as to be completely vaporized, and the concentrations thereof were adjusted to be 10, 20 ppm. After left stand for a whole day, the methyl mercaptane concentrations (B) in the bottles were measured by using a detector tube kit (manufactured by Gas-Tech Co.).

(3) Calculating the methyl mercaptane deodorizing ratio.

A value obtained by subtracting the concentration (B) of methyl mercaptane after deodorized from the concentration (A) of methyl mercaptane of when not deodorized, was divided by the concentration (A) of methyl mercaptane of when not deodorized, and was expressed as the deodorization ratio in percentage.

4. Measuring the particle size distribution of the fatty acid metal salt.

The fatty acid metal salts obtained in Examples 7 to 10 and in Comparative Examples 3 and 4 were dispersed in ethanol, and were measured for their volume-cumulative particle diameter D90 and volume-cumulative average particle diameter D50 based on the particle size distribution measuring method of the laser diffraction/scattering type by using a micro track particle size analyzer (Micro Track HRA manufactured by Nikkiso Co.).

5. Measuring the contents of unreacted substances or by-products.

Fatty acid silvers obtained in Examples 11 to 15 and in Comparative Examples 5 to 7 were heated at 900°C for 1 hour to obtain solids thereof which were then dissolved in nitric acid and were measured for their contents of unreacted substances or by-products by using an ICP emission analyzer (manufactured by Thermo-Fischer Co.). The content of the unreacted substance or the by-product was found according to,

$$
\text{Content} = \text{(Mol number of unreacted substance or by-product)/(Mol number of fatty acid silver)} \times 100
$$

6. Preparation of resin molded articles.

Fatty acid silvers obtained in Examples 11 to 15 and Comparative Examples 5 to 7 were added each in an amount of 0.5% by weight to a low-density polyethylene (manufactured by Sumitomo Kagaku Co.) which was then heat-melted in an injection-molding machine set at a temperature of 250°C to obtain the resin molded articles measuring 3.0 cm x 2.5 cm x 0.3 cm.

7. Measuring the deodorizing ratios of the resin molded articles.

Resin molded articles obtained in 6. above were hung in the 500-mL glass bottles (manufactured by GL-Science Co.) using a Naflon (registered trademark) yarn. A rotor was introduced for stirring, and the interiors of the containers were purged with nitrogen and sealed. Thereafter, by using a micro syringe, 5 $\mu$L of an aqueous solution of methyl mercaptane was added thereto dropwise so as to attain desired concentrations. The methyl mercaptane aqueous solution was stirred for 15 minutes so as to be completely vaporized. After left to stand for a whole day, the methyl mercaptane concentrations in the bottles were measured by using a detector tube kit (manufactured by Gas-Tech Co.).
The deodorizing ratio was found according to,

$$
\text{Deodorizing ratio} = \text{(Blank concentration - concentration of sample in the bottle)/(Blank concentration)} \times 100
$$

(Example 1)

There were prepared a solution (a) by dissolving 76.6 g of sodium stearate in 3000 g of water maintained at 90°C and a solution (b) by dissolving 40.3 g of silver nitrate in 600 g of water. Next, the solution (b) was thrown into the solution (a) while stirring the solution (a). After having stirred for 15 minutes from the throwing, washing was conducted to a sufficient degree by using deionized water while effecting the solid-liquid separation by filtration by means of suction. By using a hot-air drier (SPH-101 manufactured by Tabai-Espec Co.), the obtained solid was dried at 100°C for 24 hours to obtain a silver stearate having a water content of 80 ppm. Next, by using the silver stearate, a film was prepared containing the ultrafine metal particles. The obtained film was subjected to the above deodorizing test to calculate the deodorizing ratio.

(Example 2)

A silver stearate was prepared in the same manner as in Example 1 but conducting the hot-air drying at 100°C for 20 hours so that the water content was 125 ppm. A film containing ultrafine metal particles was prepared and was subjected to the deodorizing test to calculate the deodorizing ratio.

(Example 3)

A silver stearate was prepared in the same manner as in Example 1 but conducting the hot-air drying at 100°C for 18 hours so that the water content was 184 ppm. A film containing ultrafine metal particles was prepared and was subjected to the deodorizing test to calculate the deodorizing ratio.

(Example 4)

A silver stearate was prepared in the same manner as in Example 1 but conducting the drying by using the hot-air drier at 80°C for 18 hours and, thereafter, conducting the drying by using a vacuum drier (LV-120 manufactured by Tabai-Espec Co.) under the conditions of 0.1 MPa and 60°C for 8 hours so that the water content was 66 ppm. A film containing ultrafine metal particles was prepared and was subjected to the deodorizing test to calculate the deodorizing ratio.

(Example 5)

A silver stearate was prepared in the same manner as in Example 1 but drying a solution (c) obtained by dissolving 14.2 g of silver nitrate and 24.3 g of cobalt (II) nitrate hexahydrate in 600 g of water and the solution (a) of Example 1 by using the hot-air drier at 80°C for 20 hours and, thereafter, by using the vacuum drier under the conditions of 0.1 MPa and 60°C for 12 hours so that the water content was 153 ppm. A film containing ultrafine metal particles was prepared and was subjected to the deodorizing test to calculate the deodorizing ratio.

(Example 6)

A silver stearate was prepared in the same manner as in Example 5 but using a solution (d) obtained by dissolving 14.2 g of silver nitrate and 20.1 g of cupper (II) nitrate hexahydrate in 600 g of water to obtain a stearate containing silver and cupper and having a water content of 25 ppm. A film containing ultrafine metal particles was prepared and was subjected to the deodorizing test to calculate the deodorizing ratio.

(Comparative Example 1)

A silver stearate was prepared in the same manner as in Example 1 but conducting the hot-air drying at 100°C for 12 hours so that the water content was 373 ppm. A film containing ultrafine metal particles was prepared and was subjected to the deodorizing test to calculate the deodorizing ratio.

(Comparative Example 2)

A silver stearate was prepared in the same manner as in Example 1 but conducting the hot-air drying at 100°C for 6 hours so that the water content was 640 ppm. A film containing ultrafine metal particles was prepared and was subjected to the deodorizing test to calculate the deodorizing ratio.

Table 1 shows the results of Examples 1 to 6 and Comparative Examples 1 and 2.

Table 1

| | Fatty acid metal salt | Water content (ppm) | Mercaptane concentration after deodorized (10 ppm) | Deodorizing ratio (%) | Mercaptane concentration after deodorized (20 ppm) | Deocorizing ratio (%) |
|---|---|---|---|---|---|---|
| Ex. 1 | Silver stearate | 80 | 0.4 | 96 | 7 | 65 |
| Ex. 2 | Silver stearate | 125 | 3 | 70 | 12 | 40 |
| Ex. 3 | Silver stearate | 184 | 5.5 | 45 | 14.6 | 27 |
| Ex. 4 | Silver stearate | 66 | 0.4 | 96 | 7 | 65 |
| Ex. 5 | Silver stearate | 153 | 6.7 | 67 | 12 | 40 |
| Ex. 6 | Stearate containing Au, Cu | 25 | 6.7 | 67 | 10 | 50 |

(continued)

| | Fatty acid metal salt | Water content (ppm) | Mercaptane concentration after deodorized (10 ppm) | Deodorizing ratio (%) | Mercaptane concentration after deodorized (20 ppm) | Deocorizing ratio (%) |
|---|---|---|---|---|---|---|
| Comp. Ex. 1 | Silver stearate | 373 | 8 | 20 | 17.5 | 12.5 |
| Comp. Ex. 2 | Silver stearate | 640 | 9 | 10 | 19 | 5 |

The resin molded articles to which the fatty acid metal salts of Examples 1 to 6 are added, cause the methyl mercaptane concentrations to be lowered after the passage of a whole day. This means that the resin molded articles to which the fatty acid metal salts of Examples 1 to 6 are added have excellent deodorizing performance.

(Example 7)

There were prepared a solution (a) by dissolving 76.6 g of sodium stearate in 3000 g of water maintained at 90°C and a solution (b) by dissolving 40.3 g of silver nitrate in 600 g of water. Next, by using stirrer vanes of a radius of such a length as d/D = 0.42 (wherein D is a radius of a reaction vessel, d is a radius of the stirrer vanes), the solution (b) was thrown into the solution (a) while stirring the solution (a) at a rate of 700 rpm. After having stirred for 15 minutes from the throwing, washing was conducted to a sufficient degree by using deionized water while effecting the solid-liquid separation by filtration by means of suction. By using the hot-air drier (SPH-101 manufactured by Tabai-Espec Co.), the obtained solid was dried at 100°C for 24 hours to obtain a silver stearate.
The obtained silver stearate was measured for its volume-cumulative particle diameter D90, volume-cumulative average particle diameter D50 and water content. By using the silver stearate, a film containing the ultrafine metal particles was prepared.
The obtained film was subjected to the above deodorizing test to calculate the deodorizing ratio.

(Example 8)

A silver stearate was prepared in the same manner as in Example 7 but conducting the stirring at 500 rpm. A film containing ultrafine metal particles was prepared and was subjected to the deodorizing test to calculate the deodorizing ratio.

(Example 9)

A silver stearate was prepared in the same manner as in Example 7 but using stirrer vanes of a radius of such a length as d/D = 0.55, conducting the stirring at a speed of 336 rpm, and pressing the obtained solid with 0.2 MPa to obtain a cake thereof. A film containing ultrafine metal particles was prepared and was subjected to the deodorizing test to calculate the deodorizing ratio.

(Example 10)

A silver stearate was prepared in the same manner as in Example 7 but heating a solution (c) obtained by dissolving 14.2 g of silver nitrate and 20.1 g of copper (II) nitrate hexahydrate in 600 g of water by using the hot-air drier at 80°C for 20 hours and, thereafter, by using the vacuum drier (LV-120 manufactured by Tabai-Espec Co.) under the conditions of 0.1 MPa and 60°C for 12 hours to obtain a stearate containing silver and copper. A film containing ultrafine metal particles was prepared and was subjected to the deodorizing test to calculate the deodorizing ratio.

(Comparative Example 3)

A silver stearate was prepared in the same manner as in Example 7 but conducting the strring at a speed of 500 rpm, adding the solution (b) dropwise to the solution (a) at a feeding rate of 5 mL a second and conducting the hot-air drying at 100°C for 2 hours. A film containing ultrafine metal particles was prepared and was subjected to the deodorizing test to calculate the deodorizing ratio.

(Comparative Example 4)

A silver stearate was prepared in the same manner as in Example 9 but preparing a solution (b) by dissolving 40.25 g of silver nitrate in 600 g of water, conducting the pressing with 0.7 MPa and omitting the hot-air drying. A film containing ultrafine metal particles was prepared and was subjected to the deodorizing test to calculate the deodorizing ratio.

Table 2 shows the results of Examples 7 to 10 and Comparative Examples 3 and 4.

Table 2

| | Fatty acid metal salt | D50 (μm) | D90 (μ) | Water content (ppm) | Mercaptane concentration after deodorized (20 ppm) | Deocorizing ratio (%) |
|---|---|---|---|---|---|---|
| Ex. 7 | Silver stearate | 17.5 | 33.0 | 71 | 10 | 50 |
| Ex. 8 | Silver stearate | 20.9 | 39.7 | 69 | 11.2 | 44 |
| Ex. 9 | Silver stearate | 22.0 | 42.5 | 154 | 11.6 | 42 |
| Ex. 10 | Stearate containing Ag, Cu | 13.2 | 32.9 | 107 | 10.6 | 47 |
| Comp. Ex. 3 | Silver stearate | 37.2 | 82.8 | 800 | 19 | 5 |
| Comp. Ex. 4 | Silver stearate | 40.8 | 88.0 | 1500 | 19 | 5 |

The resin molded articles to which the fatty acid metal salts having D90 of not larger than 80 μm and D50 of not larger than 30 μm of Examples 7 to 10 are added, cause the methyl mercaptane concentrations to be lowered after the passage of a whole day. This means that the resin molded articles to which the fatty acid metal salts of Examples 7 to 10 are added have excellent deodorizing performance.

(Example 11)

There were prepared a solution (A) by dissolving 76.6 g of sodium stearate in 3000 g of water maintained at 90°C and a solution (B) by dissolving 40.3 g of silver nitrate in 600 g of water. Next, the solution (B) was thrown into the solution (A) while stirring the solution (A). After having stirred for 15 minutes from the throwing, washing was conducted to a sufficient degree by using 30 L of deionized water while effecting the solid-liquid separation by filtration by means of suction. By using the hot-air drier (manufactured by Tabai-Espec Co.), the drying was conducted for 12 hours and by using the vacuum drier (manufactured by Tabai-Espec Co.), the drying was, further, conducted under 0.1 mmHg at 60°C for 6 hours to obtain a silver stearate. The results of analysis showed that the silver stearate contained 0.68 mol% of sodium.

(Example 12)

A silver stearate containing 1.02 mol% of sodium was obtained by the same synthesizing method as that of Example 11 but using 15 L of water for washing.

(Example 13)

A silver stearate containing 1.70 mol% of sodium was obtained by the same synthesizing method as that of Example 11 but using 10 L of water for washing.

(Example 14)

A silver myristate containing 0.51 mol% of potassium was obtained by the same synthesizing method as that of Example 11 but using 66.5 g of potassium myristate instead of using sodium stearate.

(Example 15)

A silver myristate containing 0.85 mol% of potassium was obtained by the same synthesizing method as that of Example 14 but using 15.0 L of water for washing.

(Comparative Example 5)

A silver stearate containing 6.31 mol% of sodium was obtained by the same synthesizing method as that of Example 11 but using 1.0 L of water for washing.

(Comparative Example 6)

A silver stearate containing 9.03% of sodium was obtained by the same synthesizing method as that of Example 11 but without conducting the washing.

(Comparative Example 7)

A silver myristate containing 8.52% of potassium was obtained by the same synthesizing method as that of Example 14 but without conducting the washing.

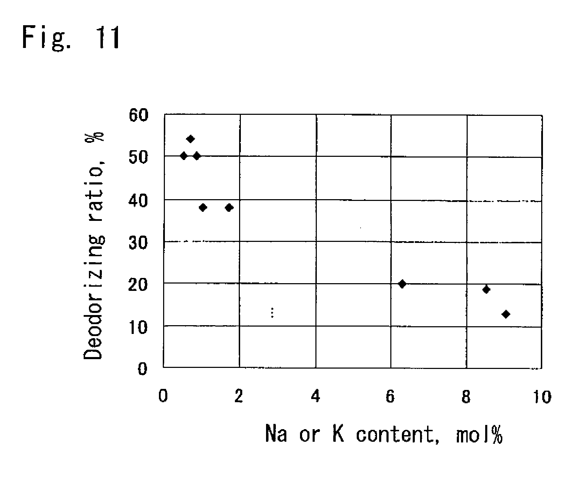

Table 3 shows the measured results of Examples 11 to 15 and Comparative Examples 5 to 7, and Fig.11 shows a relationship between the contents of sodium or potassium and the deodorizing ratios of Table 3. The resin molded articles to which the fatty acid silver salts containing small amounts of unreacted substances or by-products are added, cause the methyl mercaptane concentrations to be lowered after the passage of a whole day. This means that the resin compositions containing ultrafine metal particles formed from the fatty acid metal salt containing not more than 4.0 mol% of unreacted substances or by-products of the present invention have excellent adsorption performance.

Table 3

| | Amount of washing water (L) | Content of Na or K (mol%) | Mercaptane concentration (ppm) | Deodorizing ratio (%) |
|---|---|---|---|---|
| Blank | - | - | 20 | - |
| Ex. 11 | 30 | 0.68 | 9.3 | 54 |
| Ex. 12 | 15 | 1.02 | 12.5 | 38 |
| Ex. 13 | 10 | 1.70 | 12.5 | 38 |
| Ex. 14 | 30 | 0.51 | 10 | 50 |
| Ex. 15 | 15 | 0.85 | 10 | 50 |
| Comp. Ex. 5 | 1.0 | 6.31 | 16 | 20 |
| Comp. Ex. 6 | - | 9.03 | 17.5 | 13 |
| Comp. Ex. 7 | - | 8.52 | 16.3 | 19 |

Industrial Applicability:

Upon being used as a precursor for forming ultrafine metal particles, the fatty acid metal salt of the present invention stably forms ultrafine metal particles of an average particle diameter of 1 to 100 nm in a resin, in a coating material or in a dispersion medium to exhibit very excellent performance such as adsorbing offensively smelling substances and VOCs, antibacterial property and inactivating microproteins, and can, therefore, be utilized in a variety of industrial fields.

**Claims**

1. A fatty acid metal salt used for forming ultrafine metal particles satisfying at least one of that:

    (i) the water content is 200 ppm or less;
    (ii) the volume-cumulative particle diameter D90 is 80 $\mu$m or smaller as measured by the particle size distribution measuring method of the laser diffraction/scattering type; or
    (iii) a metal of an atomic weight of 50 to 200 is contained, and the amount of the unreacted substance or the by-product is 4.0 mol% or less when the fatty acid metal salt is formed.

2. The fatty acid metal salt according to claim 1, wherein said ultrafine metal particles have an adsorptive property and/or a property for inactivating microproteins.

3. The fatty acid metal salt according to claim 1, wherein said metal is at least the one selected from the group consisting of Cu, Ag, Au, Id, Pd, Pt, Fe, Ni, Co, Zn, Nb, Ru and Rh.

4. The fatty acid metal salt according to claim 1, wherein said metal is a combination of at least the one selected from the group consisting of Cu, Au, Id, Pd, Pt, Fe, Ni, Co, Zn, Nb, Ru and Rh, and Ag.

5. The fatty acid metal salt according to claim 1, wherein said fatty acid has 3 to 30 carbon atoms.

6. A method of producing a resin composition containing ultrafine metal particles, comprising heating the fatty acid metal salt of claim 1 at a temperature at which the fatty acid metal salt thermally decomposes in a resin but lower than a temperature at which the resin starts deteriorating so that ultrafine metal particles are formed from the fatty acid metal salt in the resin and are dispersed therein.

7. A method of producing a coating containing ultrafine metal particles, comprising heating the fatty acid metal salt of claim 1 at a temperature at which the fatty acid metal salt thermally decomposes in the coating but lower than a temperature at which the coating component starts deteriorating so that ultrafine metal particles are formed from the fatty acid metal salt in the coating and are dispersed therein.

8. A method of producing a dispersion medium containing ultrafine metal particles, comprising heating the fatty acid metal salt of claim 1 at a temperature at which the fatty acid metal salt thermally decomposes in the dispersion medium but lower than a boiling point of the dispersion medium so that ultrafine metal particles are formed from the fatty acid metal salt in the dispersion medium and are dispersed therein.

# Fig. 1

# Fig. 2

Fig. 3

Fig. 4

Fig. 5

Fig. 6

Fig. 7

Fig. 8

Fig. 9

Fig. 10

Fig. 11

| | | | |
|---|---|---|---|
| **INTERNATIONAL SEARCH REPORT** | | International application No. | |
| | | PCT/JP2009/053559 | |

**A. CLASSIFICATION OF SUBJECT MATTER**
*C07C53/126*(2006.01)i, *B22F9/30*(2006.01)i, *C07C51/02*(2006.01)i

According to International Patent Classification (IPC) or to both national classification and IPC

**B. FIELDS SEARCHED**

Minimum documentation searched (classification system followed by classification symbols)
C07C53/126, B22F9/30, C07C51/02

Documentation searched other than minimum documentation to the extent that such documents are included in the fields searched
    Jitsuyo Shinan Koho      1922-1996   Jitsuyo Shinan Toroku Koho  1996-2009
    Kokai Jitsuyo Shinan Koho  1971-2009   Toroku Jitsuyo Shinan Koho  1994-2009

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>A | Koichi AOKI, "Yang NIANJUN, Voltammetry ni yoru Shibosan Gin Nano Ryushi no Kozo Kettei", The Electrochemical Society of Japan Dai 71 Kai Taikai Koen Yoshishu, The Electrochemical Society of Japan, 24 March, 2004 (24.03.04), page 426 | 1-3,5<br>4,6-8 |
| X<br>A | B. B. BOKHONOV, Formation of Nano-Sized Silver Particles during Thermal and Photochemical Decomposition of Silver Carboxylates, Journal of Imaging Science and Technology, 2001, Vol. 45, No.3, 259-266 | 1-3,5<br>4,6-8 |

☒ Further documents are listed in the continuation of Box C.    ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
|   14 May, 2009 (14.05.09) |   26 May, 2009 (26.05.09) |

| Name and mailing address of the ISA/ | Authorized officer |
|---|---|
|   Japanese Patent Office | |
| Facsimile No. | Telephone No. |

Form PCT/ISA/210 (second sheet) (April 2007)

**INTERNATIONAL SEARCH REPORT**

| International application No. |
| --- |
| PCT/JP2009/053559 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2006-348213 A  (Tokan Material Technology Co., Ltd.), 28 December, 2006 (28.12.06), Claims; examples (Family: none) | 1-8 |
| A | JP 10-183207 A  (Tomoe Works Co., Ltd.), 14 July, 1998 (14.07.98), Claims; examples & US 6358611 B1        & EP 960675 A1 & WO 1998/026889 A1      & KR 10-2000-0057617 A | 1-8 |

Form PCT/ISA/210 (continuation of second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10183207 A
- JP 2005348213 A
- WO 200829932 A
- WO 200869034 A